# EUROPÄISCHE PATENTSCHRIFT

(11) **EP 2 349 986 B1**
(45) Veröffentlichungstag und Bekanntmachung des Hinweises auf die Patenterteilung: **24.04.2013**
(21) Anmeldenummer: 09783838.7
(22) Anmeldetag: 08.10.2009
(51) Int. Cl.: C07C 263/10, C07C 265/14

(54) **VERFAHREN ZUR HERSTELLUNG VON ISOCYANATEN**
METHOD FOR PRODUCING ISOCYANATES
PROCEDE POUR PRODUIRE DES ISOCYANATES

(30) Priorität: 15.10.2008 EP 08166624
(43) Veröffentlichungstag der Anmeldung: 03.08.2011
(73) Patentinhaber: BASF SE, 67056 Ludwigshafen (DE)
(72) Erfinder: MATTKE, Torsten, 67251 Freinsheim (DE); KNÖSCHE, Carsten, 67150 Niederkirchen (DE)
(86) Internationale Anmeldenummer: PCT/EP2009/063071
(87) Internationale Veröffentlichungsnummer: WO 2010/043532

(56) Entgegenhaltungen:
- EP-A1- 1 935 875
- EP-A1- 1 935 876

## Beschreibung

Die Erfindung geht aus von einem Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quenchraum eines Quenchs durch Zugabe eines Quenchmediums abgekühlt wird.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität, ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von Chlorwasserstoff (HCI) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 beschrieben.

Um Folgereaktionen zu vermeiden, ist es notwendig, das Reaktionsgemisch nach Reaktionsende schnell abzukühlen. Hierzu wird zum Beispiel ein Flüssigkeitsquench eingesetzt. Ein solcher Flüssigkeitsquench ist beispielsweise in EP-A 1 403 248 oder in DE-A 10 2006 058 634 beschrieben. Das Quenchmedium, das zur Kühlung zugegeben wird, weist dabei eine Temperatur auf, die im Bereich von 50 bis 200°C liegt. Durch den eingedüsten Flüssigkeitsstrom kühlt das Reaktionsgas schnell auf Temperaturen im Allgemeinen zwischen 100 und 200°C ab. Dabei entsteht ein Zwei-Phasen-Gemisch mit einer isocyanatreichen Flüssigphase und einer isocyanatarmen Gasphase. Beide werden anschließend einer gemeinsamen oder gegebenenfalls separaten Trennstufe, zum Beispiel eine Destillationsstufe zur Trennung von Chlorwasserstoff und Phosgen auf der einen Seite und Isocyanat auf der anderen Seite zugeführt.

Nachteil an den bekannten Quenchapparaten ist jedoch, dass die Wände des Quenchapparates insbesondere in der Nähe der Zuführung des Quenchmediums Temperaturen aufweisen, die unterhalb des Kondensations- beziehungsweise Desublimationspunktes des Reaktionsgases liegen. Aus diesem Grund können Teile des Reaktionsgases bereits an diesen Stellen auskondensieren beziehungsweise desublimieren. Dieses Kondensat beziehungsweise Desublimat weist an diesen Stellen eine vergleichsweise große Verweilzeit auf und kann Folgereaktionen unterliegen, die sich negativ auf die Selektivität des Gesamtprozesses auswirken.

Zudem können durch das Auskondensieren beziehungsweise Desublimieren des Reaktionsgases die Düsen, durch die das Quenchmedium zugegeben wird, verstopfen. Zudem können auch Wandablagerungen im Quenchraum entstehen.

Aufgabe der vorliegenden Erfindung ist es somit, ein Verfahren zur Herstellung von Isocyanaten durch Umsetzung der korrespondierenden Amine mit Phosgen bereitzustellen, bei dem ein Auskondensieren beziehungsweise Desublimieren des Reaktionsgases im Bereich der Zugabestellen des Quenchmediums vermieden wird.

Gelöst wird die Aufgabe durch ein Verfahren zur Herstellung von Isocyanaten durch Umsetzung der korrespondierenden Amine mit Phosgen in der Gasphase gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quenchraum eines Quenchs durch Zugabe eines Quenchmediums abgekühlt wird. Das Quenchmedium weist bei der Zugabe in den Quenchraum eine Temperatur oberhalb der Kondensationstemperatur beziehungsweise der Desublimationstemperatur des Reaktionsgases auf.

Durch die Temperatur des Quenchmediums oberhalb der Kondensationstemperatur beziehungsweise der Desublimationstemperatur des Reaktionsgases wird vermieden, dass Reaktionsgas im Bereich der Zugabe des Quenchmediums an den Zugabestellen für das Quenchmedium beziehungsweise an den Wänden des Quenchraums auskondensiert beziehungsweise desublimiert. Auf diese Weise kann vermieden werden, dass die Zugabestellen für das Quenchmedium verstopfen. Die Reinigungsintervalle für den Quenchapparat können vergrößert werden.

Zur Herstellung des lsocyanates werden das Phosgen und das Amin vorzugsweise zunächst einer Mischzone zugeführt, in der die Vermischung von Amin und Phosgen zu einem Reaktionsgemisch erfolgt. Anschließend wird das Reaktionsgemisch dem Reaktor zugeführt, in dem die Umsetzung zum Isocyanat erfolgt. Die Umsetzung von Amin und Phosgen im Reaktor erfolgt dabei in der Gasphase. Hierzu liegt der Druck im Reaktor vorzugsweise im Bereich zwischen 0,3 bis 3 bar absolut, besonders bevorzugt im Bereich von 0,8 bis 3,0 bar absolut. Die Temperatur liegt dabei vorzugsweise im Bereich von 250 bis 500°C, insbesondere im Bereich von 300 bis 480°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck des zugegebenen Amins liegt dabei vorzugsweise im Bereich zwischen 0,05 bis 3 bar absolut. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur gleichzeitigen Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Um eine Reaktion im Bereich der Mischzone zu vermeiden oder die Reaktionsgeschwindigkeit abzusenken, ist es vorteilhaft, eine turbulente Strömung in der Mischzone zu erzielen, um eine schnelle Vermischung zu erzielen. Weiterhin ist es vorteilhaft, die Temperatur in der Mischzone unterhalb der eigentlichen Reaktionstemperatur zu erhalten und das Reaktionsgemisch zwischen der Mischzone und dem Reaktor auf Reaktionstemperatur zu erhitzen. Hierzu kann zum Beispiel zwischen der Mischzone und dem Reaktor ein Diffusor eingesetzt werden.

Der Reaktor, der zur Phosgenierung des Amins zur Herstellung von Isocyanaten eingesetzt wird, ist dem Fachmann bekannt. Im Allgemeinen werden als Reaktor Rohrreaktoren eingesetzt. Im Reaktor wird das Amin mit dem Phosgen zum korrespondierenden Isocyanat und Chlorwasserstoff umgesetzt. Üblicherweise wird das Phosgen in Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Amine und Isocyanate können aliphatisch, cycloaliphatisch oder aromatisch sein.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylen-di(phenylisocyanat) (MDI), 2,4- und/oder 2,6-Toluylendiisocyanat (TDI) und 1,5- oder 1,8-Naphthyldiisocyanat (NDI).

Bevorzugte (cyclo)aliphatische Diisocyanate sind solche mit 4 bis 20 C-Atomen.

Beispiele für übliche (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendiisocyanat, 1,10-Decamethylendiisocyanat, 1,1 2-Dodecamethylendiisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(iso-cyanatocyclohexyl)methan, 1-Isocyanato-3,3,5-tri-methyl-5-(isocyanatomethyl)cyclo-hexan (Isoforondiisocyanat), 1,3- oder 1,4-Bis(isocyanatomethyl)cyclohexan, 2,4- oder 2,6-Diisocyanato-1-methylcyclohexan.

Bevorzugt sind 1,6-Diisocyanatohexan, 1-Isocyanato-3,3,5-trimethyl-5-(isocyanatomethyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5-trimethyl-5-(isocyanatomethyl)cyclohexan und 4,4'-Di(isocyanatocyclohexyl)me-than.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IPDA) und 4,4-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4'- und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Isocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Toluol, Xylol, Chlornaphtalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas unmittelbar nach der Reaktion in einem Quenchraum abgekühlt. Hierzu wird ein vorzugsweise flüssiges Quenchmedium zugegeben, das durch Verdampfung Wärme aufnimmt und zu einer schnellen Abkühlung des Reaktionsgases führt.

Um zu vermeiden, dass Reaktionsprodukte oder Reaktionsnebenprodukte im Bereich der Zugabe des Quenchmediums auskondensieren und so zum Beispiel zu einem Verstopfen der Zugabestellen des Quenchmediums führen können, weist das zugegebene Quenchmedium erfindungsgemäß eine Temperatur oberhalb der Kondensations- beziehungsweise Desublimationstemperatur des Reaktionsgases auf.

Die Zugabe des Quenchmediums erfolgt dabei vorzugsweise über mindestens eine Düse. Mehr bevorzugt erfolgt die Zugabe des Quenchmediums über mindestens zwei Düsen, wobei die Düsen vorzugsweise an gleicher axialer Position gleichmäßig verteilt in Umfangsrichtung positioniert sind. Hierdurch kann ein gleichmäßiges Zugeben des Quenchmediums realisiert werden.

Durch die Temperatur des Quenchmediums oberhalb der Kondensationstemperatur beziehungsweise der Desublimationstemperatur des Reaktionsgases wird vermieden, dass sich die Zugabepositionen, insbesondere die Düsen und auch die Wandungen des Quenchraums auf eine Temperatur unterhalb der Kondensations- beziehungsweise Desublimationstemperatur abkühlen. Auf diese Weise wird vermieden, dass sich Teile des Reaktionsgases an den Wandungen beziehungsweise den Düsen niederschlagen, auskondensieren beziehungsweise desublimieren und so zu einem Verstopfen der Düsen beziehungsweise einer Verunreinigung der Wandungen führen können.

Als Quenchmedium, das zum Abkühlen des Reaktionsgases zugegeben wird, eignet sich prinzipiell jede beliebige Flüssigkeit, die gegenüber dem im Reaktionsgas enthaltenen Komponenten inert ist. Bevorzugt wird als Quenchmedium jedoch ein gegeben-enfalls mit Halogen substituierter Kohlenwasserstoff eingesetzt. Besonders bevorzugt ist das Quenchmedium ausgewählt aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlornaphthalin, Decahydronaphthalin und Toluol. Ganz besonders bevorzugt wird als Quenchmedium Monochlorbenzol eingesetzt.

Neben den gegebenenfalls mit Halogenatomen substituierten Kohlenwasserstoffen eignet sich als Quenchmedium jedoch beispielsweise auch das Isocyanat.

Eine schnelle Abkühlung wird insbesondere dadurch erzielt, dass das Quenchmedium fein zerstäubt zugegeben wird. Hierdurch weist das Quenchmedium eine große Oberfläche auf und kann schnell die Wärme aufnehmen und damit das Reaktionsgas abkühlen.

Erfindungsgemäß wird das Quenchmedium flüssig mit einer Temperatur oberhalb der Kondensationstemperatur des Reaktionsgases zugegeben. Um eine vorzeitige Verdampfung des Quenchmediums zu vermeiden muss gegebenenfalls der Druck in der Zuleitung gegenüber dem Druck im Quenchraum erhöht sein. Die Entspannung auf den Druck des Quenchraums kann dann durch die Düsen selbst oder aber geeignete Regelorgane realisiert werden. Mit der Entspannung des Quenchmediums und Mischung mit den heißen Reaktionsgasen erfolgt eine Erwärmung und/oder eine teilweise oder vollständige Verdampfung des Quenchmediums. Die dabei aufgenommene Wärme führt zu einer Abkühlung der Reaktionsgase.

Insbesondere bei Verwendung eines Quenchmediums, das bei den Bedingungen im Quenchraum eine Siedetemperatur unterhalb der Kondensationstemperatur des Reaktionsgases aufweist, ist der Druck in den Zuleitungen gegenüber dem Druck im Quenchraum erhöht, um ein Verdampfen des Quenchmediums vor der Zugabe in den Quenchraum zu vermeiden.

Der Druck, mit dem das Quenchmedium zugegeben wird, liegt vorzugsweise im Bereich von 1 bis 20 bar, mehr bevorzugt im Bereich von 1 bis 10 bar und insbesondere im Bereich von 1 bis 8 bar.

In einer Ausführungsform der Erfindung können sich an den Quench weitere Stufen zur Abkühlung des Reaktionsgases anschließen. In den einzelnen Stufen zur Abkühlung erfolgt dabei jeweils eine weitere Abkühlung des Reaktionsgases, bis zum Erreichen der gewünschten Endtemperatur, mit der das Reaktionsgas beispielsweise einer nachfolgenden Aufarbeitung zugeführt wird.

In einer Ausführungsform ist zumindest eine der sich an den Quench anschließenden Stufen zur Abkühlung des Reaktionsgases ein weiterer Quench.

So ist es zum Beispiel möglich, das den Quench und die sich gegebenenfalls daran anschließenden Stufen zur Abkühlung verlassende Reaktionsgas vorzugsweise bei Temperaturen von mehr als 130°C mit einem Lösungsmittel zu waschen. Als Lösungsmittel eignen sich zum Beispiel die gleichen Stoffe, die auch als Quenchmedium eingesetzt werden können.

Bei der Wäsche wird das Isocyanat selektiv in die Waschlösung überführt. Anschließend werden das verbleibende Gas und die erhaltene Waschlösung bevorzugt mittels Rektifikation in Isocyanat, Lösungsmittel, Phosgen und Chlorwasserstoff aufgetrennt.

Das Waschen des den Reaktor verlassenden Gasgemisches erfolgt vorzugsweise in einem Waschturm, wobei aus dem gasförmigen Gasgemisch das gebildete Isocyanat durch Kondensation in einem inerten Lösungsmittel abgetrennt wird, während überschüssiges Phosgen, Chlorwasserstoff und gegebenenfalls das Inertmedium die Aufarbeitungsvorrichtung gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Die Wäsche kann in jeder beliebigen, dem Fachmann bekannten Vorrichtung durchgeführt werden. So eignen sich zum Beispiel Rührbehälter oder andere herkömmliche Apparaturen, beispielsweise Kolonnen oder Mixer-Settler-Apparaturen.

Das Waschen und die Aufarbeitung des den Reaktor verlassenden Reaktionsgases erfolgt dabei im Allgemeinen wie beispielsweise im WO-A 2007/028715 beschrieben.

### Beispiel

In einer Anlage zur Herstellung von Isocyanat werden ungefähr 1,8 kg/h TDA phosgeniert. Der im Reaktor entstehende Reaktionsgasstrom von 15,95 kg/h weist eine Temperatur von 458°C auf und enthält 15,7 Gew.-% TDI, 71,2 Gew.-% Phosgen und 13,1 Gew.-% HCI. Der Taupunkt des Reaktionsgases liegt bei 183°C. Das Reaktionsgas wird in einer Quenchapparatur mit Monochlorbenzol abgekühlt. Hierzu wird das als Quenchmedium eingesetzte Monochlorbenzol flüssig bei einem Druck von 6 bar auf 200°C erhitzt und dem Quenchraum über eine Sprühdüse zugeführt. Im Quenchraum herrscht ein Druck von 2 bar absolut. Die sich einstellende Mischtemperatur im Quenchraum beträgt ungefähr 157°C. Dabei fallen ungefähr 2,15 kg/h einer an TDI-reichen Flüssigkeit mit einem Gehalt von ungefähr 64 Gew.-% TDI und 33,8 kg/h einer TDI-armen Gasphase mit einem TDI-Gehalt von ca. 3,3 Gew.-% an.

Aufgrund der Zuführtemperatur des Quenchmediums von 200°C im Vergleich zur Kondensationstemperatur des Reaktionsgases von 183°C kann das frühzeitige Auskondensieren von Teilen des Reaktionsgases und damit die Bildung von Ablagerungen im Bereich der Zuführung des Quenchmediums vermieden werden.

## Patentansprüche

1. Verfahren zur Herstellung von Isocyanaten durch Umsetzung der korrespondierenden Amine mit Phosgen in der Gasphase gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quenchraum eines Quenchs durch Zugabe eines Quenchmediums abgekühlt wird, **dadurch gekennzeichnet, dass** das Quenchmedium bei der Zugabe in den Quenchraum eine Temperatur oberhalb der Kondensationstemperatur beziehungsweise der Desublimationstemperatur des Reaktionsgases aufweist.

2. Verfahren gemäß Anspruch 1, **dadurch gekennzeichnet, dass** das Amin und das Phosgen in der Gasphase umgesetzt werden.

3. Verfahren gemäß Anspruch 1 oder Anspruch 2, **dadurch gekennzeichnet, dass** das Amin und das Phosgen gasförmig zugegeben werden.

4. Verfahren gemäß einem der Ansprüche 1 bis 3, **dadurch gekennzeichnet, dass** das Quenchmedium über mindestens eine Düse zugegeben wird.

5. Verfahren gemäß einem der Ansprüche 1 bis 4, **dadurch gekennzeichnet, dass** das Quenchmedium ein gegebenenfalls mit Halogen substituierter Kohlenwasserstoff ist.

6. Verfahren gemäß einem der Ansprüche 1 bis 5, **dadurch gekennzeichnet, dass** das Quenchmedium ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophtalat, Tetrahydrofuran, Dimethylformamid, Xylol, Chlornaphthalin, Decahydronaphthalin und Toluol.

7. Verfahren gemäß einem der Ansprüche 1 bis 6, **dadurch gekennzeichnet, dass** das Quenchmedium flüssig mit einer Temperatur oberhalb der Kondensationstemperatur des Reaktionsgases zugegeben wird.

8. Verfahren gemäß einem der Ansprüche 1 bis 7, **dadurch gekennzeichnet, dass** das Quenchmedium unter den Bedingungen im Quenchraum eine Siedetemperatur unterhalb der Kondensationstemperatur des Reaktionsgases aufweist und der Druck in den Zuleitungen des Quenchmediums gegenüber dem Druck im Quenchraum erhöht ist, um ein Verdampfen des Quenchmediums vor der Zugabe in den Quenchraum zu vermeiden.

9. Verfahren gemäß einem der Ansprüche 1 bis 7, **dadurch gekennzeichnet, dass** das Quenchmedium unter Zulaufbedingungen eine Siedetemperatur oberhalb der Kondensationstemperatur des Reaktionsgases aufweist.

10. Verfahren gemäß einem der Ansprüche 1 bis 9, **dadurch gekennzeichnet, dass** das Quenchmedium mit einem Druck im Bereich von 1 bis 20 bar zugegeben wird.

11. Verfahren gemäß einem der Ansprüche 1 bis 10, **dadurch gekennzeichnet, dass** das Quenchmedium unmittelbar nach der Zugabe entspannt, wodurch die Temperatur des Quenchmediums unter die Kondensationstemperatur des Reaktionsgases sinkt.

12. Verfahren gemäß einem der Ansprüche 1 bis 11, **dadurch gekennzeichnet, dass** das Quenchmedium nach Zugabe in den Quenchraum zumindest teilweise verdampft.

13. Verfahren gemäß einem der Ansprüche 1 bis 12, **dadurch gekennzeichnet, dass** sich an den Quench weitere Stufen zur Abkühlung des Reaktionsgases anschließen.

14. Verfahren gemäß Anspruch 13, **dadurch gekennzeichnet, dass** mindestens eine der sich an den Quench anschließenden Stufen zur Abkühlung des Reaktionsgases ein weiterer Quench ist.

## Claims

1. A process for preparing isocyanates by reacting the corresponding amines with phosgene in the gas phase, optionally in the presence of an inert medium, in which the amine and the phosgene are first mixed and converted to the isocyanate in a reactor, and in which a redaction gas which comprises isocyanate and hydrogen chloride and leaves the reactor is cooled in a quench space of a quench by adding a quench medium, wherein the quench medium of addition to the quench space has a temperature above the condensation temperature or the desublimation temperature of the redaction gas.

2. The process according to claim 1, wherein the amine and the phosgene are reacted in the gas phase.

3. The process according to claim 1 or claim 2, wherein the amine and the phosgene are added in gaseous form.

4. The process according to any one of claims 1 to 3, wherein the quench medium is added via at least one nozzle.

5. The process according to any one of claims 1 to 4, wherein the quench medium is an optionally halogen-substituted hydrocarbon.

6. The process according to any one of claims 1 to 5, wherein the quench medium is selected from the group consisting of monochlorobenzene, dichlorobenzene, trichlorobenzene, hexane, benzene, 1,3,5-trimethylbenzene, nitrobenzene, anisole, chlorotoluene, o-dichlorobenzene, diethyl isophthalate, tetrahydrofuran, dimethylformamide, xylene, chloronaphthalene, decahydronaphthalene and toluene.

7. The process according to any one of claims 1 to 6, wherein the quench medium is added in liquid form with a temperature above the condensation temperature of the redaction gas.

8. The process according to any one of claims 1 to 7, wherein the quench medium, under the conditions in the quench space, has a boiling temperature below the condensation temperature of the reaction gas, and the pressure in the feed lines oaf the quench medium is elevated compared to the pressure in the quench space in order to prevent evaporation of the quench medium before addiction to the quench space.

9. The process according to any one of claims 1 to 7, wherein the quench medium, under feed condition, has a boiling temperature above the condensation temperature of the redaction gas.

10. The process according to any one of claims 1 to 9, wherein the quench medium is added with a pressure in the range from 1 to 20 bar.

11. The process according to any one of claims 1 to 10, wherein the quench medium is decompressed immediately after the addition, which lowers the temperature of the quench medium below the condensation temperature of the reaction gas.

12. The process according to any one of claims 1 to 11, wherein the quench medium evaporates at least partly after addition to the quench space.

13. The process according to any one of claims 1 to 12, wherein the quench is followed by further stages for cooling the reaction gas.

14. The process according to claim 13, wherein at least one of the stages for cooling the reaction gas which follow the quench is a further quench.

## Revendications

1. Procédé pour la production d'isocyanates par mise en réaction des amines correspondances avec du phosgène dans la phase gazeuse éventuellement en présence d'un milieu inerte, dans lequel d'abord on mélange l'amine et le phosgène, puis on les convertit en l'isocyanate dans un réacteur, en refroidissant un gaz de réaction contenant de l'isocyanate et du chlorure d'hydrogène, quittant le réacteur, dans une chambre de refroidissement d'un refroidisseur à injection, par addition d'un milieu de refroidissement, **caractérisé en ce que** le milieu de refroidissement présente lors de l'introduction dans la chambre de refroidissement une température supérieure à la température de condensation ou à la température de désublimation du gaz de réaction.

2. Procédé selon la revendication 1, **caractérisé en ce que** l'amine et le phosgène sont mis en réaction dans la phase gazeuse.

3. Procédé selon la revendication 1 ou la revendication 2, **caractérisé en ce que** l'amine et le phosgène sont ajoutés sous forme de gaz.

4. Procédé selon l'une quelconque des revendications 1 à 3, **caractérisé en ce qu'**on introduit le milieu de refroidissement au moyen d'au moins une buse.

5. Procédé selon l'une quelconque des revendications 1 à 4, **caractérisé en ce que** le milieu de refroidissement est un hydrocarbure éventuellement substitué par halogène.

6. Procédé selon l'une quelconque des revendications 1 à 5, **caractérisé en ce que** le milieu de refroidissement est choisi dans le groupe constitué par le monochlorobenzène, le dichlorobenzène, le trichlorobenzène, l'hexane, le benzène, le 1,3,5-triméthylbenzène, le nitrobenzène, l'anisole, le chlorotoluène, l'o-dichlorobenzène, l'isophtalate de diéthyle, le tétrahydrofurane, le diméthylformamide, le xylène, le chloronaphtalène, le décahydronaphtalène et le toluène.

7. Procédé selon l'une quelconque des revendications 1 à 6, **caractérisé en ce que** le milieu de refroidissement est introduit à l'état liquide, à une température supérieure à la température de condensation du gaz de réaction.

8. Procédé selon l'une quelconque des revendications 1 à 7, **caractérisé en ce que** le milieu de refroidissement présente dans les conditions régnant dans la chambre de refroidissement une température d'ébullition inférieure à la température de condensation du gaz de réaction et la pression dans les conduits amenant le milieu de refroidissement est élevée par rapport à la pression régnant dans la chambre de refroidissement, afin d'éviter une évaporation du milieu de refroidissement avant l'introduction dans la chambre de refroidissement.

9. Procédé selon l'une quelconque des revendications 1 à 7, **caractérisé en ce que** le milieu de refroidissement présente dans les conditions d'alimentation une température d'ébullition supérieure à la température de condensation du gaz de réaction.

10. Procédé selon l'une quelconque des revendications 1 à 9, **caractérisé en ce que** le milieu de refroidissement est introduit sous une pression dans la plage de 1 à 20 bars.

11. Procédé selon l'une quelconque des revendications 1 à 10, **caractérisé en ce que** le milieu de refroidissement est défendu immédiatement après l'introduction, de sorte que la température du milieu de refroidissement s'abaisse au-dessous de la température de condensation.

12. Procédé selon l'une quelconque des revendications 1 à 11, **caractérisé en ce que** le milieu de refroidissement s'évapore au moins partiellement après introduction dans la chambre de refroidissement.

13. Procédé selon l'une quelconque des revendications 1 à 12, **caractérisé en ce que** d'autres étapes pour le refroidissement du gaz de réaction font suite au refroidissement par injection.

14. Procédé selon la revendication 13, **caractérisé en ce qu'**au moins l'une des étapes pour le refroidissement du gaz de réaction, faisant suite au refroidissement par injection, est un autre refroidissement par injection.
